# EUROPEAN PATENT APPLICATION

(11) **EP 3 735 994 A1**
(43) Date of publication of application: **11.11.2020**
(21) Application number: 19173428.4
(22) Date of filing: 09.05.2019
(51) Int. Cl.: A61M 1/00, A61M 1/06

(54) **ELECTRIC BREAST PUMP**

(71) Applicant: Koninklijke Philips N.V., 5656 AG Eindhoven (NL)
(72) Inventor: HESEN, Robertus Gerardus Johannes Antonius, 5656 AE Eindhoven (NL)
(74) Representative: de Haan, Poul Erik

(57) **Abstract**

An electrical breast pump (10) is provided with a vacuum source (100) having a vacuum pump (120) with an electrical motor (121), and a controller (110) controlling an operation of the vacuum pump (120) in a stimulation mode and an extracting mode for milk extraction. In the extraction mode each pumping cycle comprises a pumping period (PP) and an aerate period (AP). The controller (110) controls a motor supply voltage for the electrical motor (121) of the vacuum pump (120). During each pumping period (PP) in a first stage, an initial voltage value is set, in a second stage, the initial voltage value is kept constant for a first time period, in a third stage, the voltage is increased with a first slope, in a fourth stage, the voltage is increased with a second slope and in a fifth stage, the voltage reaches its nominal value.

## Description

### FIELD OF THE INVENTION

The present invention relates to an electric breast pump and a method of controlling an electric breast pump.

### BACKGROUND OF THE INVENTION

Electric breast pumps are used by women in order to express milk from their breast to feed the milk to their babies at a desired time. A breast pump typically comprises an expression kit with a funnel into which the breast of the user is placed. The expression kit also comprises a bottle into which the expressed milk can flow. The expression kit is coupled to a vacuum source to enable the expression of milk. The vacuum source provides a vacuum which is applied to the breast in the funnel to extract milk. The breast pump may comprise two operating modes, namely a stimulation mode and an extracting mode. During the stimulation mode, the milk ejection reflex is stimulated. When the breast pump is activated, a vacuum source like a vacuum pump is activated and generates a vacuum. The vacuum pump is typically activated by an electrical motor. During the extracting mode, a pumping cycle of the vacuum pump comprises a pumping period followed by an aerate period. During the pumping period, the vacuum pump is activated while it is not activated during the aerate period. As a user of the breast pump is typically immobile during the extraction of milk, a reduced time for extracting the milk would be appreciated.

US 5,676,525 discloses an electrical breast pump with a vacuum pump and a controller controlling the operation of the vacuum pump.

### SUMMARY OF THE INVENTION

It is an object of the invention to provide a breast pump which enables a fast and efficient milk extraction.

According to an aspect of the invention, a breast pump is provided which comprises a vacuum source with a vacuum pump operated by an electrical motor. Furthermore, a controller is provided which is adapted to control the vacuum pump and in particular the electrical motor of the vacuum pump in a stimulation mode and an extracting mode for milk extraction. In the extraction mode, each pumping cycle comprises a pumping period and an aerate period. The controller is adapted to control a motor supply voltage during the pumping period in five stages. In the first stage, an initial voltage value is set. In the second stage, the initial voltage is kept substantially constant for a first time period. In the third stage, the voltage is increased with a first slope. In the fourth stage, the voltage is increased with a second slope and in the fifth stage, the voltage reaches its nominal value.

With the different stages of the motor supply voltage, the motor supply voltage value is reached faster while the current of the electrical motor does not exceed a threshold value. In other words, the motor accelerates as fast as possible within the current limitation.

According to an embodiment, the second slope is smaller than the first slope.

According to a further embodiment, the controller uses pulse width modulation to control the value of the motor supply voltage.

According to an embodiment, the controller uses pulse width modulation to control the value of the motor supply voltage. Hence, the controller can control the voltage in a easily controllable fashion.

According to an embodiment, the first time period during the second stage is smaller than the time period during the third and fourth stage.

According to an embodiment, in the second stage, the current is increased wherein in the third stage, the current is further increased. In the fourth stage, the current is substantially constant.

According to an aspect of the invention, a method of controlling an electrical breast pump is provided. The breast pump comprises a vacuum source having a vacuum pump with an electrical motor. The operation of the vacuum pump is controlled in a stimulation mode and an extracting mode for milk extraction. In the extraction mode, each pumping cycle of the vacuum pump comprises a pumping period and an aerate period. A motor supply voltage for the electrical motor of the vacuum pump is controlled. In a first stage, an initial voltage value is set. In a second stage, the initial voltage is kept constant for a first time period. In a third stage, the voltage is increased with a first slope. In a fourth stage, the voltage is increased with a second slope. In a fifth stage, the voltage reaches its nominal value.

It should be noted the motor for the breast pump must be started for each pump period and stopped again after the pump period. Accordingly, the pump will be activated and deactivated a great number of times. Furthermore, any improvement of the starting procedure of the motor while not producing an undue current would significantly reduce the time required for extracting milk from a breast of user of the breast pump and thus reduce the time required by the user.

According to an aspect of the invention a computer program for operating a breast pump is provided. The computer program comprising program code means for causing an electrical breast pump to carry out the steps of the method of operating a breast pump as defined above, when the computer program is run on a computer controlling the breast pump.

Other aspects of the invention are described in the dependent claims. Further advantages and embodiments of the invention will now be elucidated with reference to the drawings.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a block diagram of a breast pump according to an embodiment,
Fig. 2 shows a graph depicting a voltage and current profile for a breast pump according to Fig. 1,
Fig. 3 shows a graph of a measured voltage profile of a breast pump, and
Fig. 4 shows a graph depicting a measured current of a breast pump according to Fig. 1

### DETAILED DESCRIPTION OF EMBODIMENTS

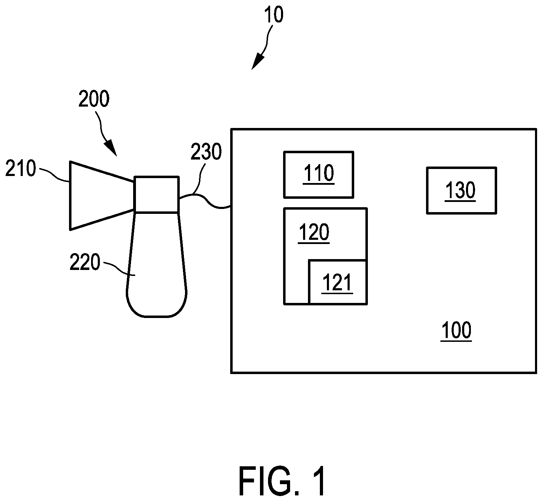

Fig. 1 shows a block diagram of a breast pump according to an embodiment. The breast pump 10 comprises an expression kit 200 and a vacuum source 100. The expression kit 200 comprises at least one funnel 210 for receiving a breast of a user, and a bottle 220 for collecting expressed milk. The expression kit 200 is coupled to the vacuum source 100 via a vacuum conduit 230.

The vacuum source 100 comprises a controller 110, a vacuum pump 120 with a motor 121. Furthermore, the vacuum source 100 comprises a power supply 130.

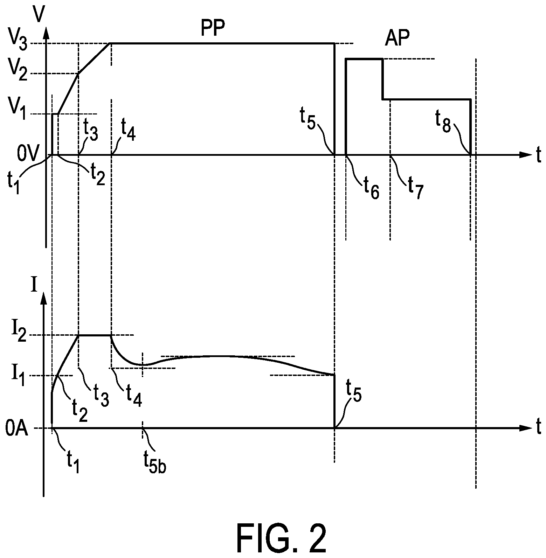

Fig. 2 shows a graph depicting a voltage and current profile for a breast pump according to Fig. 1. During operation, the controller 110 controls the operation of the vacuum pump 120. In particular, the breast pump 10 has a stimulation mode and an extraction mode for stimulating the milk ejection reflex. When the breast of a user is placed into the funnel 210 and when the vacuum source 100 is activated, the vacuum pump 120 creates a vacuum which is supplied via the vacuum conduit 230 to the breast placed in the funnel 210.

After the stimulation mode, the extraction mode is activated. In the extraction mode, a pumping cycle is continuously repeated. During each pumping cycle, a pumping period PP and an aerate period AP are present. During the pumping period PP, the controller 110 controls a motor supply voltage for the motor 121 of the vacuum pump 120. This control can be performed in at least five stages. During a first stage at the point of time t1, an initial voltage value V1 is set or applied to the motor 121 of the vacuum pump 120. Then the voltage is kept constant at V1 until t2. After t2, the voltage will increase from V1 to V2 at a first slope until t3. After t3, the voltage will further increase from V2 to V3 but with a second slope until t4. At t4, the voltage reaches its nominal value V2 and is substantially constant until t5, when the voltage is reduced to zero.

At t1, the current will increase due to initial voltage V1 until t2. Between t2 and t3, the current will further increase from I1 with a slope. Between t3 and t4, the current is substantially constant and after t4, the current will decrease until it reaches t5b. After the decrease of the current, the current may increase slightly again and then decrease until it reaches t5. After t5, it is zero as the voltage is also zero.

With the above described five stages, the time the voltage reaches its nominal value is decreased while it is ensured that the current flowing through the motor 121 does not exceed a threshold value.

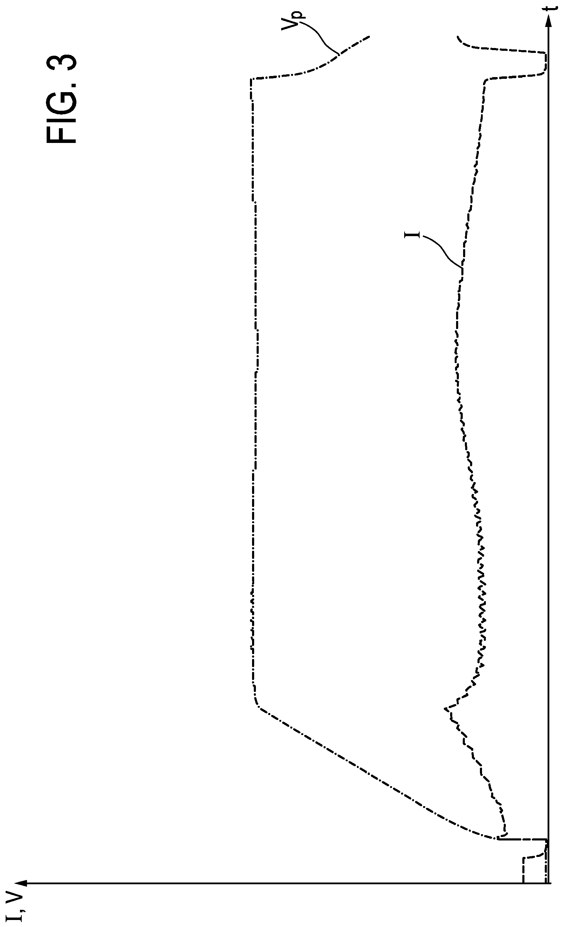

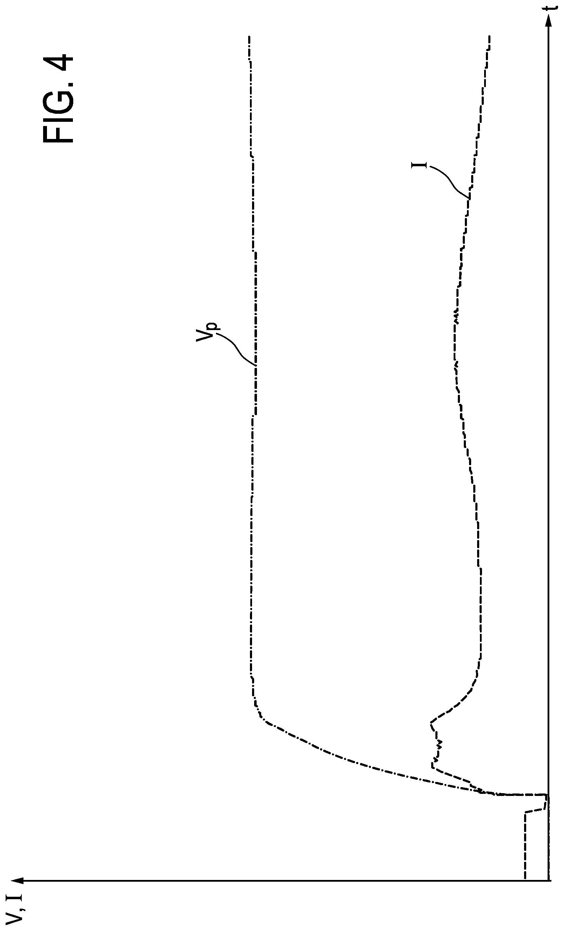

Fig. 3 shows a graph of a measured voltage profile of a breast pump according to Fig. 1 and Fig. 4 shows a graph depicting a measured current of a breast pump according to Fig. 1.

Limiting the voltage between t1 and t2 to the initial voltage value V1 is advantageous as this will avoid that the current value exceeds a threshold value. Although this time period between t1 and t2 will increase the time required during a pump period PP, this particular voltage profile is however advantageous in view of limiting the current in the motor 121. The immediate increase of the voltage to its nominal value would result in an undue increase of the corresponding current.

According to an embodiment, the voltage ramp up is divided into three stages, namely a stage with a constant voltage, a stage with a voltage increase with a first slope and a stage with a voltage increase with a second slope, the second slope being less or smaller than the first slope. This is advantageous as a fast speed up of the nominal rotating frequency of the electric motor 121 can be achieved within the limits of the maximum constraints for the power supply current. Thus, while not exceeding the current limit from the power supply, a maximum acceleration or a maximum rotation speed of the motor for the vacuum pump 120 is achieved. The time interval of the first stage with the substantially constant voltage is significantly smaller than the time period of the other two stages during which the voltage is increased.

This three stage voltage ramp up is in view of the current flow through the motor improved over a direct setting of the nominal voltage at the start of the motor, i.e. providing a very steep voltage ramp. Such a steep voltage ramp would lead to a significant and undesired increase of the current through the motor. At the beginning of the start up or ramp up of the voltage, the motor coil will be loaded with the maximum possible current thus generating the maximum electromagnetic field and thus having an improved start or acceleration. However, as mentioned, this undue increase of the current is not advantageous in view of the lifetime of the motor. In fact, such a procedure can significantly reduce the lifetime of the motor and other components of the vacuum pump.

In Fig. 3, only the pumping period is depicted with a start up of the pump motor with a normal ramp up. The voltage profile of Fig. 3 is achieved by the controller 110 by using a pulse width modulated output signal. The pulse width modulated voltage of the pump Vp as shown in Fig. 3 is filtered. The frequency of the pulse width modulated output signal must be higher than the electrical motor constant.

In Fig. 4, the pump voltage Vp and an average current through the modulated power stage for the pump is depicted.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. Electrical breast pump (10), comprising
a vacuum source (100) having a vacuum pump (120) with an electrical motor (121), and
a controller (110) configured to control an operation of the vacuum pump (120) in a stimulation mode and an extracting mode for milk extraction,
wherein in the extraction mode each pumping cycle comprises a pumping period (PP) of the vacuum pump (120) and an aerate period (AP) during which the vacuum pump (120) is inactive,
wherein the controller (110) is adapted to control a motor supply voltage for the electrical motor (121) of the vacuum pump (120),
wherein during each pumping period (PP) in a first stage, an initial voltage value is set, in a second stage, the initial voltage value is kept constant for a first time period, in a third stage, the voltage is increased with a first slope, in a fourth stage, the voltage is increased with a second slope and in a fifth stage, the voltage reaches its nominal value.

2. Electrical breast pump (10) according to claim 1, wherein
the second slope is smaller than the first slope.

3. Electrical breast pump (10) according to claim 1 or 2, wherein
the controller (110) uses a pulse width modulation to control the value of the motor supply voltage.

4. Electrical breast pump (10) according to one of the claims 1 to 3, wherein
the first time period during the second stage is smaller than the time periods during the third and fourth stage.

5. Electrical breast pump (10) according to any one of the claims 1 to 4, wherein
in the second stage, the current is increased, wherein in the third stage, the current is further increased, wherein in the fourth stage, the current is substantially constant.

6. Electrical breast pump (10) according to any one of the claims 1 to 5, further comprising
an expression kit (200) having at least one funnel (210) adapted for receiving a breast of a user, a bottle (220) adapted to collect extracted milk and a port for a vacuum conduit (230) through which the expression kit is coupled to the vacuum source (100).

7. Method of controlling an electrical breast pump (10) having a vacuum source (100) with a vacuum pump (120) and an electrical motor (121), comprising the steps of:
controlling an operation of the vacuum pump (120) in a stimulation mode and an extraction mode for milk extraction, wherein in the extraction mode each pumping cycle comprises a pumping period (PP) and an aerate period (AP),
controlling a motor supply voltage for the electrical motor (121) of the vacuum pump (120),
setting an initial voltage in a first stage,
keeping the initial voltage constant for a first time period in a second stage,
increasing the voltage with a first slope in a third stage,
increasing the voltage with a second slope in a fourth stage,
wherein in a fifth stage, the voltage has reached its nominal value.

8. A computer program for operating a breast pump, the computer program comprising program code means for causing an electrical breast pump as defined in claim 1 to carry out the steps of the method of operating a breast pump as defined in claim 7, when the computer program is run on a computer controlling the breast pump.
